(19) Europäisches Patentamt
European Patent Office
Office européen des brevets

(11) Veröffentlichungsnummer: **0 044 417**
**B1**

(12) # EUROPÄISCHE PATENTSCHRIFT

(45) Veröffentlichungstag der Patentschrift: **23.01.85**

(51) Int. Cl.[4]: **C 07 D 249/12**, C 07 D 403/06, C 07 D 403/12

(21) Anmeldenummer: **81104768.7**

(22) Anmeldetag: **22.06.81**

(54) **Triazolidin-3,5-dion-Formaldehyd-Amin-Kondensate und Verfahren zu ihrer Herstellung.**

(30) Priorität: **21.07.80 DE 3027582**

(43) Veröffentlichungstag der Anmeldung: **27.01.82 Patentblatt 82/4**

(45) Bekanntmachung des Hinweises auf die Patenterteilung: **23.01.85 Patentblatt 85/4**

(84) Benannte Vertragsstaaten: **DE FR GB IT**

(56) Entgegenhaltungen:
**DE - A - 2 936 842**
**FR - A - 1 330 401**

(73) Patentinhaber: **BAYER AG, Konzernverwaltung RP Patentabteilung, D-5090 Leverkusen 1 Bayerwerk (DE)**

(72) Erfinder: **Rottmaier, Ludwig, Bergstrasse 85, D-5068 Odenthal (DE)**
Erfinder: **Merten, Rudolf, Dr., Berta-von Suttner-Strasse 55, D-5090 Leverkusen (DE)**

**EP 0 044 417 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft gegebenenfalls substituierte Triazolidin-3,5-dion-Formaldehyd-Amin-Kondensate und ein Verfahren zu ihrer Herstellung.

Es wurden neue Triazolidin-3,5-dion-Formaldehyd-Amin-Kondensate der Formel

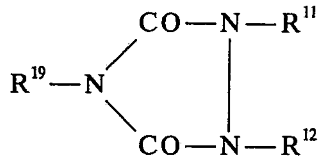

(I)

in der

$R^{11}$ und $R^{12}$ unabhängig voneinander Wasserstoff, $C_2-C_8$-Alkylcarbonyl oder die Gruppe

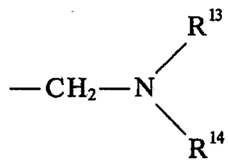

bedeuten, wobei

$R^{13}$ für Wasserstoff, $C_1-C_8$-Alkyl, $C_5-C_6$-Cycloalkyl, Benzyl oder Phenyl steht,

$R^{14}$ $C_1-C_8$-Alkyl, $C_2-C_8$-Alkenyl, Benzyl, Phenyl, einen ein oder mehrere Stickstoff-, Sauerstoff- oder Schwefelatome enthaltenden heterocyclischen Rest, $C_2-C_8$-Alkylcarbonyl, $C_3-C_8$-Alkenylcarbonyl oder $C_2-C_8$-Alkoxycarbonyl bedeutet und in der weiterhin

$R^{13}$ und $R^{14}$ gemeinsam mit dem Stickstoffatom, das sie substituieren, einen gegebenenfalls weitere Stickstoff-, Sauerstoff- oder Schwefelatome enthaltenden Stickstoffheterocyclus bedeuten können, und

$R^{19}$ für $C_1-C_{20}$-Alkyl, $C_5-C_6$-Cycloalkyl, Benzyl, Phenyl, $C_2-C_8$-Alkylcarbonyl oder für die Gruppe

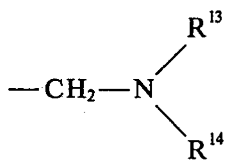

steht, in der

$R^{13}$ und $R^{14}$ die angegebene Bedeutung haben

oder für die Gruppe

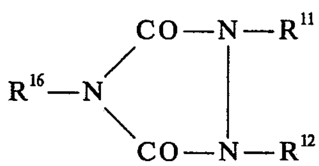

steht, in der

$R^{16}$ $C_1-C_{12}$-Alkylen, Phenylen, Toluylen oder eine der Gruppen

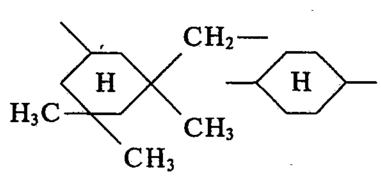

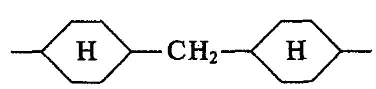

oder

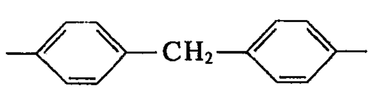

bedeutet, und

$R^{11}$ und $R^{12}$ die obengenannte Bedeutung haben,

oder für die Gruppe

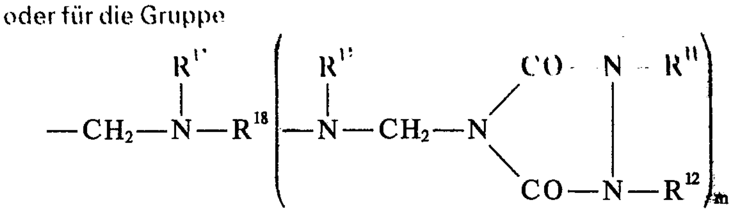

steht, in der

$R^{11}$ und $R^{12}$ die obengenannte Bedeutung haben,
$R^{17}$ für Wasserstoff, $C_1-C_4$-Alkyl, Cyclohexyl, Benzyl, Phenyl, einen ein oder mehrere Stickstoff-, Sauerstoff- oder Schwefelatome enthaltenden heterocyclischen Rest oder die Gruppe

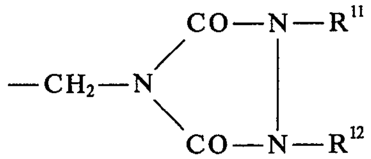

steht,

$R^{18}$ ein $(m+1)$-wertiger aliphatischer Rest $(C_2-C_8)$, Cyclohexanrest, Benzolrest oder ein ein oder mehrere Stickstoff-, Sauerstoff- oder Schwefelatome enthaltender heterocyclischer Rest ist,
m die Zahl 0, 1 oder 2 bedeutet, wobei für den Fall, daß $m=1$ ist, $R^{18}$ auch die Carbonylgruppe sein kann,

und wobei die Reste $R^{11}$, $R^{12}$ und $R^{19}$ mindestens eine aus der Formaldehyd-Amin-Kondensation hervorgegangene Gruppe

$>N-CH_2-N<$

enthalten, gefunden.

Als Alkyl sei ein geradkettiger oder verzweigter aliphatischer Rest mit 1 bis 8, bevorzugt 1 bis 4, Kohlenstoffatomen genannt, wie Methyl, Ethyl, Propyl, Isopropyl, Butyl, Isobutyl, Hexyl und Octyl.

Als Alkenyl sei ein geradkettiger oder verzweigter ungesättigter Kohlenwasserstoffrest mit 2 bis 8, besonders bevorzugt 2 bis 4, Kohlenstoffatomen genannt, wie Vinyl, Propenyl, Butenyl, Hexenyl und Octenyl.

Als Cycloalkyl sei ein cycloaliphatischer Rest mit 5 oder 6, bevorzugt mit 6, Kohlenstoffatomen genannt, wie Cyclopentyl und Cyclohexyl.

Als heterocyclischer Rest sei ein neben Kohlenstoffatomen ein oder mehrere Stickstoff-, Sauerstoff- oder Schwefelatome enthaltender Rest genannt. Für den Fall, daß ein solches Ringsystem mehrere Heteroatome enthält, können dies auch verschiedene Heteroatome sein. Beispiele hierfür sind die von folgenden Heterocyclen abgeleiteten Reste: Pyrolidin, Piperidin, Pyrazolidin, Imidazolidin, Oxazolidin, Thiazolidin, Piperazin, Morpholin, Thiomorpholin, Hydantoin, 5,5-Dimethylhydantoin, Parabansäure, Barbitursäure oder Cyanursäure.

Als Alkylcarbonyl sei beispielsweise ein an einer $\alpha$-ständigen Carbonylgruppe sitzender aliphatischer, gesättiger Kohlenwasserstoffrest genannt, wobei die Gesamtzahl der Kohlenstoffatome einschließlich der Carbonylgruppe 2 bis 8, bevorzugt 2 bis 4, beträgt. Beispiele hierfür sind: Acetyl, Propionyl, Butyryl und Hexylcarbonyl.

Als Alkenylcarbonyl sei beispielsweise ein an einer $\alpha$-ständigen Carbonylgruppe sitzender olefinischer Rest genannt, wobei die Gesamtzahl der Kohlenstoffatome einschließlich der Carbonylgruppe 3 bis 8, bevorzugt 3 oder 4, beträgt. Beispiele hierfür sind: Acrylyl, Methacrylyl, Crotonyl, bevorzugt Acrylyl und Methacrylyl.

Als Alkoxycarbonyl sei beispielsweise ein an einer $\alpha$-ständigen Carbonylgruppe sitzender Alkoxyrest bezeichnet, wobei die Gesamtzahl der Kohlenstoffatome 2 bis 8, bevorzugt 2 bis 4, beträgt. Beispiele hierfür sind: Methoxycarbonyl, Ethoxycarbonyl, Propoxycarbonyl, Butoxycarbonyl und Hexyloxycarbonyl.

Für den Fall, daß zwei Reste gemeinsam mit dem Stickstoffatom, das sie substituieren, einen

Stickstoffheterocyclus bedeuten können, der gegebenenfalls Stickstoff, Sauerstoff oder Schwefel als Heteroatome enthält, seien beispielsweise die folgenden, derart gebildeten Ringsysteme genannt: Pyrolidin, Pyrazolidin, Imidazolidin, Oxazolidin, Thiazolidin, Triazolidin, Tetrazolidin, Thiodiazolidin, Piperidin, Piperazin, Morpholin, Thiomorpholin. Weitere Beispiele sind heterocyclische Vierringe oder Siebenringe, wie Azacyclobutan oder Azacycloheptan.

m bedeutet erfindungsgemäß die Zahl 0, 1 oder 2, vorzugsweise 1 oder 2.

Alle genannten Reste können ihrerseits ein- oder mehrfach, bevorzugt ein- oder zweifach, besonders bevorzugt einfach, durch Halogen, Hydroxy, $C_1-C_4$-Alkoxy, Cyano, Amino, $C_1-C_4$-Alkylamino, Bis($C_1-C_4$-alkyl)-amino, $C_2-C_4$-Alkoxycarbonyl oder $C_1-C_4$-Alkyl, das geradkettig oder verzweigt sein kann, substituiert sein.

Besonders bevorzugt sind Triazolidin-3,5-dion-Formaldehyd-Amin-Kondensate der Formel

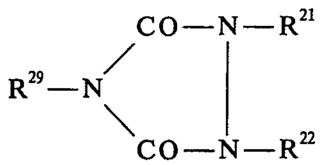

in der

$R^{21}$ und $R^{22}$ unabhängig voneinander Wasserstoff, $C_2-C_4$-Alkylcarbonyl oder die Gruppe

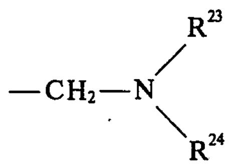

bedeuten, in der

$R^{23}$ für Wasserstoff, $C_1-C_4$-Alkyl und

$R^{24}$ für $C_1-C_4$-Alkyl, einen ein oder mehrere Stickstoff-, Sauerstoff- oder Schwefelatome enthaltenden heterocyclischen Rest, $C_2-C_4$-Alkylcarbonyl, $C_3-C_4$-Alkenyl-carbonyl stehen und wobei weiterhin

$R^{23}$ und $R^{24}$ gemeinsam mit dem Stickstoffatom, das sie substituieren, einen gegebenenfalls weitere Stickstoff-, Sauerstoff- oder Schwefelatome enthaltenden Stickstoffheterocyclus bilden können, und

$R^{29}$ $C_1-C_4$-Alkyl, Cyclohexyl, Benzyl, Phenyl, $C_2-C_4$-Alkylcarbonyl oder die Gruppe

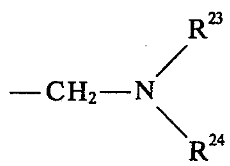

bedeutet, in der

$R^{23}$ und $R^{24}$ die oben angegebene Bedeutung haben,

wobei die Reste $R^{21}$, $R^{22}$ und $R^{23}$ mindestens eine aus der Formaldehyd-Amin-Kondensation hervorgegangene Gruppe

$$>N-CH_2-N<$$

enthalten.

Bevorzugt sind weiterhin Triazolidin-3,5-dion-Formaldehyd-Amin-Kondensate der Formel

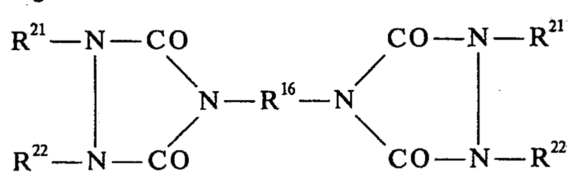

(III)

in der

$R^{21}$ und $R^{22}$ den obengenannten Bedeutungsumfang haben und
$R^{16}$ $C_1$–$C_{12}$ Alkylen, Phenylen, Toluylen oder eine der Gruppen

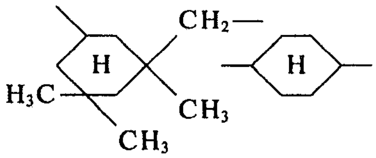

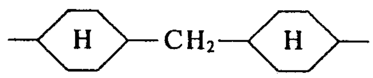

oder

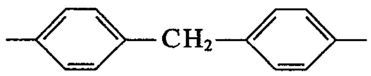

bedeutet, und wobei die Reste $R^{21}$ und $R^{22}$ mindestens eine aus der Formaldehyd-Amin-Kondensation hervorgegangene Gruppe

$>N-CH_2-N<$

enthalten.

Als Alkylen seien beispielsweise zweiwertige aliphatische Reste mit 1 bis 12, bevorzugt 1 bis 8, besonders bevorzugt 2 bis 6, Kohlenstoffatome, genannt, wie Methylen, Ethylen, Trimethylen, Tetramethylen, Hexamethylen oder Octamethylen.

Bevorzugt sind weiterhin Triazolidin-3,5-dion-Formaldehyd-Amin-Kondensate der Formel

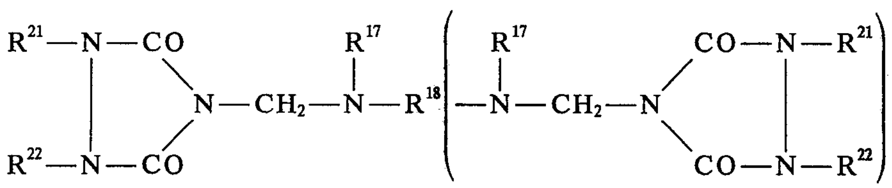

(IV)

in der

$R^{21}$ und $R^{22}$ den obengenannten Bedeutungsumfang haben,
$R^{17}$ für Wasserstoff, $C_1-C_4$-Alkyl, Cyclohexyl, Benzyl, Phenyl, einen ein oder mehrere Stickstoff-, Sauerstoff- oder Schwefelatome enthaltenden heterocyclischen Rest oder die Gruppe

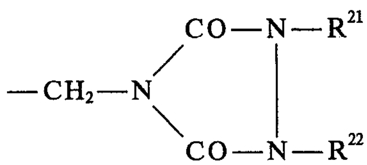

steht, in der

$R^{21}$ und $R^{22}$ den obengenannten Bedeutungsumfang haben,

$R^{18}$ ein (m+1)-wertiger aliphatischer Rest ($C_2-C_8$), Cyclohexanrest, Benzolrest oder ein ein oder mehrere Stickstoff-, Sauerstoff- oder Schwefelatome enthaltender heterocyclischer Rest ist und
m die Zahl 0, 1 oder 2 bedeutet, wobei für den Fall, daß m = 1 ist, $R^{18}$ auch die Carbonylgruppe sein kann

und wobei die Reste $R^{21}$, $R^{22}$, $R^{17}$ und $R^{18}$ mindestens eine aus der Formaldehyd-Amin-Kondensation hervorgegangene Gruppe

$>N-CH_2-N<$

enthalten.

Es wurde weiterhin ein Verfahren zur Herstellung der erfindungsgemäßen Triazolidin-3,5-dion-Formaldehyd-Amin-Kondensate gefunden, das dadurch gekennzeichnet ist, daß man Triazolidin-3,5-dione der Formel

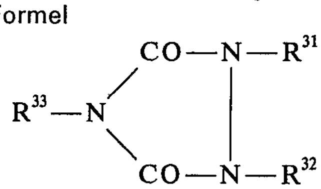

(V)

in der

$R^{31}$ und $R^{32}$ unabhängig voneinander Wasserstoff oder $C_2-C_8$-Alkylcarbonyl bedeuten und
$R^{33}$ Wasserstoff, Alkyl ($C_1-C_{20}$), Cycloalkyl ($C_5-C_6$), Benzyl, Phenyl, Alkylcarbonyl ($C_2-C_8$) oder die Gruppe

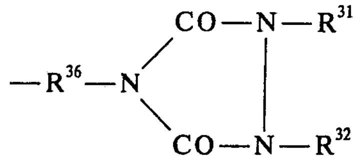

bedeutet, in der

$R^{31}$ und $R^{32}$ die obengenannte Bedeutung haben,
$R^{36}$ $C_1-C_{12}$-Alkylen, Phenylen, Toluylen oder eine der Gruppen

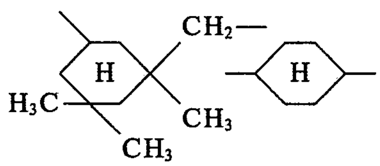

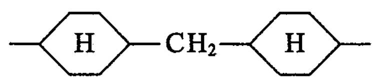

oder

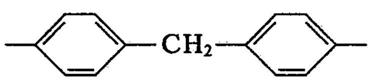

bedeutet, wobei mindestens einer der Reste $R^{31}$, $R^{32}$ und $R^{33}$ Wasserstoff bedeutet, mit Formaldehyd und Stickstoffverbindungen der Formel

$$H-X \qquad (VI)$$

in der

X für

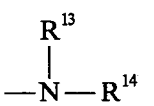 oder 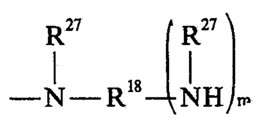

steht, worin

$R^{13}$ für Wasserstoff, $C_1-C_8$-Alkyl, $C_5-C_6$-Cycloalkyl, Benzyl oder Phenyl steht,
$R^{14}$ $C_1-C_8$-Alkyl, $C_2-C_8$-Alkenyl, Benzyl, Phenyl, einen ein oder mehrere Stickstoff-, Sauerstoff- oder Schwefelatome enthaltenden heterocyclischen Rest, $C_2-C_8$-Alkylcarbonyl, $C_3-C_8$-Alkenylcarbonyl oder $C_2-C_8$-Alkoxycarbonyl bedeutet und in der weiterhin
$R^{13}$ und $R^{14}$ gemeinsam mit dem Stickstoffatom, das sie substituieren, einen gegebenenfalls weitere Stickstoff-, Sauerstoff- oder Schwefelatome enthaltenden Stickstoffheterocyclus bedeuten können, und
$R^{18}$ ein (m+1)-wertiger aliphatischer Rest ($C_2-C_8$), Cyclohexanrest, Benzolrest oder ein ein oder mehrere Stickstoff-, Sauerstoff- oder Schwefelatome enthaltender heterocyclischer Rest ist und
m die Zahl 0, 1 oder 2 bedeutet, wobei für den Fall, daß m=1 ist, $R^{18}$ auch die Carbonylgruppe sein kann, und
$R^{27}$ Wasserstoff, $C_1-C_4$-Alkyl, Cyclohexyl, Benzyl, Phenyl oder einen ein oder mehrere Stickstoff-, Sauerstoff- oder Schwefelatome enthaltenden heterocyclischen Rest bedeutet,

bei Temperaturen von 10 bis 180°C, gegebenenfalls in Gegenwart eines Katalysators und gegebenenfalls in Gegenwart eines Lösungsmittels umsetzt.

Der Bedeutungsumfang der Substituenten der genannten Formeln ist übereinstimmend mit dem weiter oben offenbarten Bedeutungsumfang.

Die erfindungsgemäß einzusetzenden Triazolidin-3,5-dione können nach verschiedenen Verfahren hergestellt werden.

So können entsprechende Amine mit Hydrazodicarbonamid (Verfahren 1) oder mit 1,2,4-Triazolidin-3,5-dion (Verfahren 2) bei 150 bis 280°C in Gegenwart oder Abwesenheit eines Lösungsmittels wie N-Methylpyrrolidon oder eines Lösungsmittelgemisches bei Drücken von 50 mbar bis 5 mbar, gegebenenfalls in Gegenwart eines sauren oder basischen Katalysators wie Alkoholate oder tert. Amin, unter Abspaltung von Ammoniak zu den Ausgangs-1,2,4-triazolidin-3,5-dionen umgesetzt werden.

Eine weitere Möglichkeit für die Herstellung der Ausgangs-1,2,4-triazolidin-3,5-dione besteht darin, daß man entsprechende N-monosubstituierte Hydrazodicarbonamide unter den Bedingungen, wie vorstehend für die Verfahren 1 und 2 angegeben, unter Ammoniakabspaltung zu den Ausgangs-1,2,4-triazolidin-3,5-dionen umsetzt. Die N-monosubstituierten Hydrazodicarbonamide können nach bekannten Verfahren aus Semicarbazid und Isocyanaten erhalten werden.

Der Formaldehyd kann im erfindungsgemäßen Verfahren sowohl gasförmig eingeleitet werden oder in Form einer wäßrigen handelsüblichen Lösung zudosiert werden, die beispielsweise bis zu 65 Gew.-% Formaldehyd, bevorzugt bis zu 40 Gew.-% Formaldehyd enthält, als auch in polymerer Form, wie als Trioxan oder Paraformaldehyd in die Reaktionsmischung eingesetzt werden.

Als Beispiele für die Stickstoffverbindungen für das erfindungsgemäße Verfahren seien genannt:

Amine, wie Methylamin, Ethylamin, Propylamin, Butylamin, Hexylamin, Dimethylamin, Diethylamin, Dipropylamin, Dibutylamin, Methyl-butyl-amin, Bis-(hydroxyethyl)-amin, Bis-(hydroxypropyl)-amin, Cyclohexylamin, N-Methyl-cyclohexylamin, Anilin, N-Methyl-anilin, Piperidin, Piperazin, Morpholin oder Thiomorpholin, Pyrrolidin, Indol, Carbazol, Pyrazol, 2-Methyl-imidazol, Benzimidazol oder 1,2,4-Triazol. Bevorzugt werden sekundäre Amine eingesetzt:

Amide, wie Acetamid, Propionamid, Buttersäureamid, 2-chlorpropionamid, Valerianamid, Benzamid, Acetanilid, Acrylamid oder Methacrylamid:

Cyclische Amide, wie 2-Pyrrolidon, $\varepsilon$-Caprolactam, Parabansäure, Bisparabansäuren der allgemeinen Formel

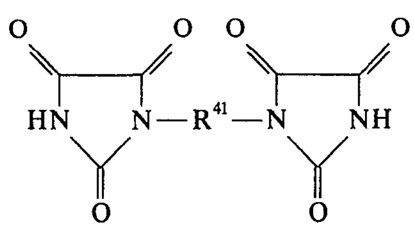

(VII)

in der

$R^{41}$ für $C_2-C_{10}$-Alkylen, $C_4-C_{10}$-Cycloalkylen oder $C_6-C_{14}$-Arylen, bevorzugt $C_2-C_4$-Alkylen, Cyclohexylen oder Phenylen steht,

oder Hydantoine der allgemeinen Formel

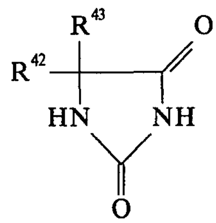

(VIII)

in der

$R^{42}$ und $R^{43}$ für Alkyl, Cycloalkyl oder Aryl stehen, wobei $R^{42}$ und $R^{43}$ auch gemeinsam eine Trimethylen-, Tetramethylen-, Pentamethylen-, Hexamethylen- oder Heptamethylen-Gruppe bilden können.

Als cyclische Amide seien weiterhin Bis-Hydantoine, die über die 1,1- oder 3,3-Stellung verknüpft sein können, Barbitursäure und cyclische Imide, wie Bernsteinsäureimid oder Phthalsäureimid, genannt.

Als Beispiele für Stickstoffverbindungen seien aliphatische, cycloaliphatische, araliphatische, aromatische oder heterocyclische Polyamine genannt, wie Ethylendiamin, Trimethylendiamin, Tetramethylendiamin, an den Stickstoffatomen alkylsubstituierte Diamine der genannten Art, Phenylendiamin, Melamin, 4,6-Diamino-2-phenyl-1,3,5-triazin, 1,2-Bis-(4,6-diamino-1,3,5-triazin-2-yl)-ethan, 1,4-Bis-(4,6-diamino-1,3,5-triazin-2-yl)-butan, 1,6-Bis- und 1,3,6-Tris-(4,6-diamino-1,3,5-triazin-2-yl)-hexan.

Das erfindungsgemäße Verfahren wird bei einer Temperatur von 10 bis 180°C, bevorzugt 20 bis 160°C durchgeführt. Häufig verläuft die Reaktion des erfindungsgemäßen Verfahrens, insbesondere bei Verwendung eines geeigneten Katalysators exotherm, so daß es zweckmäßig ist, die Reaktion bei

einer tieferen Temperatur innerhalb des genannten Bereiches zu beginnen und mit oder ohne Kühlung bei einer höheren Temperatur innerhalb des genannten Bereiches zu Ende zu führen. Für den Fall, daß — wie weiter unten erläutert — die Reaktion mehrstufig ausgeführt wird, kann es erforderlich sein, daß die erste Stufe bei einer niedrigeren Temperatur im genannten Bereich und die zweite Stufe bei einer höheren Temperatur im genannten Bereich durchgeführt wird.

Beim Einsatz niedrigsiedender Reaktionspartner sowie zur Vermeidung von Formaldehydverlusten kann es erforderlich sein, unter erhöhtem Druck zu arbeiten. Hierfür sei beispielsweise ein Bereich von 1 bis 8, bevorzugt 1 bis 5 bar, genannt.

Das erfindungsgemäße Verfahren kann ohne oder mit Lösungsmitteln durchgeführt werden. Die Durchführung ohne Lösungsmittel ist dann möglich, wenn in der Schmelze der Reaktionspartner gearbeitet wird oder wenn ein Reaktionspartner, beispielsweise die Stickstoffverbindung, flüssig ist und die anderen Reaktionspartner ganz oder teilweise löst.

Für den Fall, daß in Gegenwart eines Lösungsmittels gearbeitet wird, seien hierfür polare Lösungsmittel genannt, die mit den Reaktionspartnern nicht reagieren. Beispiele hierfür sind: Ether, wie Dibutylether, Ethylenglykoldimethylether, Dioxan oder Tetrahydrofuran, Ester, wie Essigsäurebutylester oder Glykolmonomethyletheracetat, N,N-dialkylierte Amide, wie Dimethylformaid, Dimethylacetamid oder N-Methylpyrrolidon, Sulfone, wie Dimethylsulfon oder Tetramethylensulfon, sowie Wasser und Mischungen aus Wasser und solchen Lösungsmitteln der beispielhaften Aufzählung, die mit Wasser mischbar sind. Nach beendeter Reaktion, beispielsweise zu einer besseren Aufarbeitung des Reaktionsgemisches, etwa durch Kristallisation oder Extraktion, können gegebenenfalls weitere Lösungsmittel, wie beispielsweise aliphatische oder aromatische Kohlenwasserstoffe, wie Ligroin, Cyclohexan, Toluol, Xylol, chlorierte Kohlenwasserstoffe, wie Methylenchlorid, Chloroform, Chlorbenzol, Alkohole, wie Ethanol oder Butanol oder Ketone, wie Aceton oder Butanon, zugesetzt werden. In vielen Fällen kann Wasser als Lösungsmittel Verwendung finden, da hierbei die gewünschte Verbindung auskristallisiert oder durch Zugabe von Fällungsmitteln, wie Ethanol oder Aceton kristallisiert werden kann. In solchen Fällen kann vorteilhaft das Formaldehyd als wäßrige Formalinlösung eingesetzt werden. Diese Verfahrensvariante ist daher bevorzugt.

Für den Fall, daß in Gegenwart eines Lösungsmittels gearbeitet wird, sei für dieses eine Menge von 25 bis 1000, bevorzugt 100 bis 600 Gew.-% Lösungsmittel, bezogen auf die Gesamtmenge der Reaktionspartner, genannt.

Das erfindungsgemäße Verfahren kann sowohl einstufig als auch zweistufig durchgeführt werden. Beim einstufigen Verfahren kann beispielsweise das Triazolidin-3,5-dion und die Stickstoffverbindung, gegebenenfalls in einem der genannten Lösungsmittel vorgelegt werden. Anschließend wird der Formaldehyd zudosiert. Hierfür wird in einer bevorzugten Ausführungsform wäßriger Formaldehyd verwendet. Hierbei wird zunächst eine exotherme Reaktion beobachtet und sodann die Reaktion durch Erwärmen auf die gewünschte Reaktionstemperatur zu Ende gebracht. Als Mengenverhältnisse zwischen dem Triazolidin-3,5-dion, der Sticksstoffverbindung und dem Formaldehyd sei beispielsweise für den Fall, daß jede vorhandene NH-Gruppe des Triazolidindions umgesetzt werden soll, je 0,8 bis 3 Mol, bevorzugt je 0,8 bis 1,5 Mol, besonders bevorzugt je 0,9 bis 1,1 Mol, Formaldehyd und Stickstoffverbindung pro Äquivalent NH-Gruppe genannt. Falls jedoch eine oder zwei NH-Gruppen nicht umgesetzt werden sollen, muß der Überschuß an Formaldehyd und Stickstoffverbindung begrenzt sein, als Menge für diesen Fall sei beispielsweise je 0,9 bis 1,1 Mol Formaldehyd und Stickstoffverbindung pro Äquivalent NH-Gruppe genannt.

Das Reaktionsprodukt kann nach üblichen Methoden, beispielsweise durch Kristallisation, aufgearbeitet und gereinigt werden.

Bei der zweistufigen Durchführung des erfindungsgemäßen Verfahrens kann in zwei Varianten gearbeitet werden. Bei der ersten Variante wird zunächst aus dem Triazolidin-3,5-dion und Formaldehyd die entsprechende Methylolverbindung gebildet und diese dann mit der Stickstoffverbindung weiter umgesetzt. Als eine zweite Variante kann jedoch auch zunächst aus der Stickstoffverbindung und Formaldehyd die hierzu gehörende Methylolverbindung gebildet werden, die dann anschließend mit dem Triazolidin-3,5-dion weiter umgesetzt wird.

Die einzelnen beschriebenen Teilschritte des erfindungsgemäßen Verfahrens und die einstufige Variante des erfindungsgemäßen Verfahrens können sowohl mit als auch ohne Verwendung von Katalysatoren durchgeführt werden. Die Verwendung von Katalysatoren erniedrigt im allgemeinen die Reaktionstemperatur und verkürzt im allgemeinen die Reaktionszeiten, so daß die Verwendung eines geeigneten Katalysators bevorzugt ist.

Für die als erster Teilschritt mögliche Umsetzung der Triazolidin-3,5-dione mit Formaldehyd können beispielsweise basische Katalysatoren verwendet werden. Als solche seien beispielhaft genannt: tertiäre Amide oder quaternäre Ammoniumsalze, wie Triethylamin, Tri-n-butylamin, Triethanolamin, N,N-Dimethylanilin, Tetramethylammoniumchlorid, Alkali- und Erdalkalihydroxide, wie Natriumhydroxid, Kaliumhydroxid oder Bariumhydroxid, basisch reagierende Alkali- oder Erdalkalisalze, wie Natriumtetraborat, Natriumaluminat, Lithiumcarbonat, Natriumcarbonat, Kaliumcarbonat oder Bariumcarbonat, oder Alkoholate, wie Natriummethylat. Als Menge des Katalysators sei beispielsweise eine solche von 0,001 bis 5 Gew.-%, bevorzugt 0,05 bis 2 Gew.-%, bezogen auf das Gesamtgewicht der Reaktionspartner, genannt.

Für den Fall, daß der Formaldehyd in Form der polymeren Formen, wie Trioxan oder Paraformaldehyd, eingesetzt wird, sind jedoch auch saure Katalysatoren geeignet. Als solche seien beispielsweise genannt: Mineralsäuren, wie Salzsäure, Phosphorsäure oder Schwefelsäure, Sulfonsäuren, wie p-Toluolsulfonsäure oder organische Säuren, wie Oxalsäure, Essigsäure, Ameisensäure oder Benzoesäure.

Für den Fall, daß als erste Stufe einer zweistufigen Verfahrensvariante die Umsetzung der Stickstoffverbindung mit Formaldehyd durchgeführt wird, kann diese ebenfalls in Gegenwart eines basischen Katalysators durchgeführt werden. Als basische Katalysatoren und als deren Menge für diese Verfahrensvariante können die oben beschriebenen eingesetzt werden.

Der Formaldehyd reagiert in der ersten Stufe einer zweistufigen Verfahrensvariante mit einem an einem Stickstoffatom gebundenen Wasserstoff in dem Triazolidin-3,5-dion oder in der Stickstoffverbindung unter Ausbildung einer Methylolgruppe. Der Formaldehyd wird hierbei in einer Menge von 0,8 bis 1,5, bevorzugt 0,9 bis 1,1 Mol pro Äquivalent des umzusetzenden, an einem Stickstoffatom sitzenden Wasserstoffs eingesetzt. Weitere, etwa vorhandene N—H-Gruppen, zu deren Umsetzung kein Formaldehyd in dem Reaktionsgemisch vorhanden ist, bleiben als solche erhalten. Sollen alle vorhandenen N—H-Gruppen zu Methylolgruppen umgesetzt werden, so ist über die obengenannte Menge Formaldehyd hinaus ein Überschuß an Formaldehyd möglich. Dieser Überschuß, beispielsweise 0,1 bis 2 Mol über die genannte Menge hinaus, kann die Reaktion in der ersten Stufe einer zweistufigen Verfahrensvariante beschleunigen. Er muß jedoch zur Vermeidung störender Nebenreaktionen vor der zweiten Stufe weitgehend wieder entfernt werden.

Wird in der zweiten Stufe einer zweistufigen Verfahrensvariante zu der vorgebildeten Triazolidin-3,5-dion-methylolverbindung die Stickstoffverbindung eingesetzt oder wird in der zweiten Stufe einer zweistufigen Verfahrensvariante zu der vorgebildeten Methylolverbindung der Stickstoffverbindung das Triazolidin-3,5-dion zugesetzt, kann hierbei ein basischer oder saurer Katalysator Verwendung finden, wie er bereits oben beschrieben ist. Wird daher die erste Stufe einer solchen zweistufigen Verfahrensvariante unter Benutzung eines Katalysators durchgeführt, so braucht in der Regel in der zweiten Reaktionsstufe kein zusätzlicher Katalysator zugesetzt zu werden. Die Menge der in der zweiten Stufe zugesetzten Stickstoffverbindung zur vorgebildeten Triazolidin-3,5-dion-Methylolverbindung bzw. des zugesetzten Triazolidin-3,5-dions zur vorgebildeten Methylolverbindung der Stickstoffverbindung beträgt beispielsweise 0,8 bis 1,5 Mol, bevorzugt 0,9 bis 1,1 Mol, bezogen auf jede vorgebildete Methylolgruppe. Für den Fall, daß die Stickstoffverbindung in der zweiten Stufe zur vorgebildeten Triazolidin-3,5-dion-Methylolverbindung zugesetzt wird, kann auch ein Überschuß über die obengenannte Menge verwendet werden, beispielsweise 0,1 bis 2 Mol über die bereits genannte Menge. Ein solcher Überschuß an Stickstoffverbindung kann beispielsweise als Lösungsmittel oder als zusätzliches Lösungsmittel dienen. Die Anwendung eines solchen Überschusses und seine Menge richten sich im allgemeinen nach den Erfordernissen der Aufarbeitbarkeit des Reaktionsgemisches.

Bei den zweistufigen Verfahrensvarianten des erfindungsgemäßen Verfahrens ist diejenige Variante bevorzugt, bei der zunächst die Triazolidin-3,5-dion-Methylolverbindung gebildet wird und in einer zweiten Stufe die Stickstoffverbindung zugesetzt wird. Bei dieser Variante wird die erste Reaktionsstufe im unteren Teil des oben beschriebenen Temperaturbereiches, beispielsweise bei Raumtemperatur, durchgeführt und die zweite Stufe im oberen Teil dieses Temperaturbereiches beispielsweise bei 50 bis 180° C, vorzugsweise bei 80 bis 160° C, durchgeführt.

Die erfindungsgemäßen Triazolidin-3,5-dion-Formaldehyd-Amin-Kondensate sind wertvolle Hilfsstoffe zur Modifizierung von polymeren Substanzen. So können sie beispielsweise zur Herstellung und zur Modifizierung von Melamin-, Harnstoff- und Phenolharzen eingesetzt werden. Für diesen Einsatz eignen sich die erfindungsgemäßen Stoffe sowohl als Einzelindividuen als auch als Gemische verschieden hoch methylolierter und verschieden hoch kondensierter Stoffe. Ferner können die erfindungsgemäßen Triazolidin-3,5-dion-Formaldehyd-Amin-Kondensate als Flammschutzmittel, beispielsweise in thermoplastischen Polyamidformmassen, eingesetzt werden.

## Beispiele

Wenn nicht anders angegeben, bedeuten Prozente Gewichtsprozente und Teile Gewichtsteile.

### Beispiel 1

a) Herstellung von

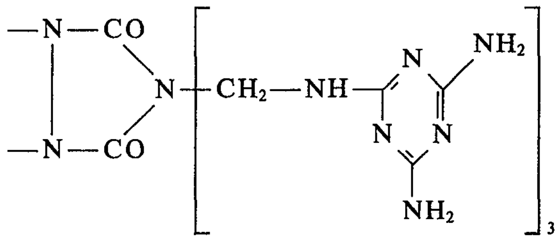

3,03 kg Triazolidin-3,5-dion werden unter Rühren zu 9,75 kg einer 37%igen wäßrigen Formaldehydlösung, die 10 g Borax enthält, gegeben und auf 100°C erwärmt. Die entstandene klare Lösung wird 90 Minuten bei 100°C gerührt und durch Abdestillieren des Wassers im Wasserstrahlvakuum eingeengt, wobei der überschüssige Formaldehyd ebenfalls entfernt wird. Die abgekühlte hellgelbe viskose Flüssigkeit, hauptsächlich Trishydroximethyl-triazolidin-3,5-dion, wird bei Raumtemperatur in 90 l entionisiertem Wasser gelöst, mit 11,34 kg Melamin versetzt und langsam bis zum Rückfluß des Wassers erwärmt. Die entstandene Mischung wird zwei Stunden bei Rückfluß gerührt, bei 90°C abgesaugt und mit heißem Wasser gewaschen. Nach dem Trocknen bei 100°C und 30 mbar werden 14,55 kg Produkt erhalten, dessen obige Formel mittels IR-Spektrum und der Elementaranalyse bestätigt wird.

berechnet für $C_{14}H_{21}N_{21}O_2$:
C 32,7, H 4,08, N 57,1%
gefunden
C 32,9, H 4,2, N 56,5%
Fp. >260°C

b) 85 Gew.-% Polyamid-6,6 mit einer relativen Viskosität (gemessen an einer Lösung aus 1 g Polyamid in 100 ml m-Kresol bei 25°C) von 2,96 werden in einem Doppelwellenextruder bei den für Polyamid-6,6 üblichen Bedingungen mit 15 Gew.-% des nach a) hergestellten Mannichkondensates in der Schmelze vermischt. Der abgezogene Strang wird gekühlt, granuliert und getrocknet. Anschließend wird das Granulat auf einer Spritzgießmaschine A 270 der Firma Arburg zu Probekörnern der Abmessung 127 × 12,7 × 1,6 mm gespritzt.

Diese Probekörper werden 24 Stunden bei 23°C und 50% rel. Luftfeuchte gelagert und ergeben bei der Brandprüfung, bei der die Nachbrennzeiten aus 10 Beflammungen bestimmt werden, eine Nachbrennzeit von 11 Sekunden, während die Prüfkörper ohne Mannichkondensat eine Nachbrennzeit von 32 Sekunden aufweisen.

Beispiel 2

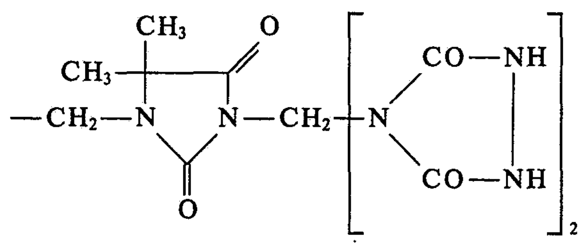

163 g einer 37%igen Formaldehydlösung, welche 2 g Borax enthält, werden mit 128 g 5,5-Dimethylhydantoin auf 80°C erwärmt und 2 Stunden bei 80°C gerührt. Die entstandene klare Lösung wird abgekühlt und nach Zugabe von 202 g Triazolidin-3,5-dion 3 Stunden bei Rückfluß gerührt. Die eingeengte Lösung wird in wenig Ethanol gelöst und von wenig unlöslichem Rückstand befreit. Die ethanolische Lösung wird durch Aufheizen auf 160°C vom Lösungsmittel und vom Restwasser befreit. Es werden 335 g eines spröden Stoffes mit harzartigem Aussehen erhalten, der überwiegend aus dem Stoff obiger Formel besteht und dessen Struktur mittels IR- und NMR-Spektrum sowie der Elementaranalyse bewiesen wird.

berechnet für $C_{11}H_{14}N_8O_6$:
C 37,3, H 3,95, N 31,6%
gefunden
C 37,0, H 3,8, N 31,2%

Beispiel 3

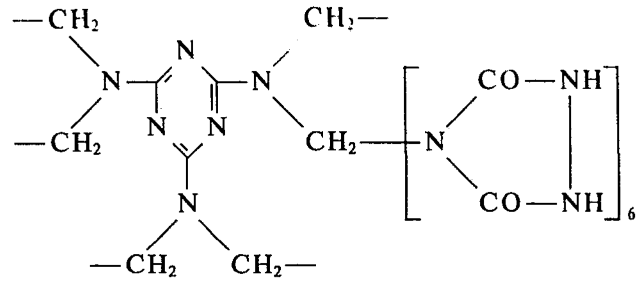

30,6 g Hexamethylolmelamin, hergestellt nach Sorensen, Campbell, Präparative Methoden der Polymeren Chemie, Verlag Chemie 1962, und 60,6 g Triazolidin-3,5-dion in 500 ml Wasser werden auf 95°C erwärmt und 2,5 Stunden bei 95°C gerührt. Nach dem Abkühlen auf Raumtemperatur wird der Niederschlag abgesaugt und mit Wasser gewaschen. Es werden 61 g getrocknetes Produkt erhalten, das überwiegend aus dem Stoff obiger Formel besteht und dessen Struktur mittels IR- und NMR-Spektrum sowie der Elementaranalyse bewiesen wird.

berechnet für $C_{21}H_{24}N_{24}O_{12}$:
C 31,3, H 2,99, N 41,7%
gefunden
C 31,1, H 3,2, N 41,6%

Beispiel 4

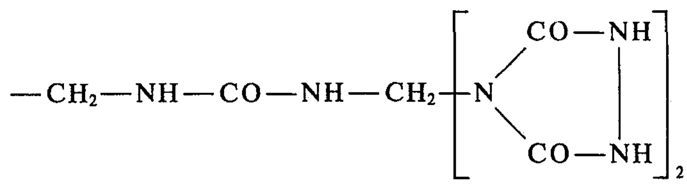

60 g Dimethylolharnstoff werden in 100 ml Wasser gelöst, mit 10%iger Natronlauge auf pH 8 gestellt, mit 101 g Triazolidin-3,5-dion versetzt und 3 Stunden bei 100°C gerührt. Die abgekühlte Lösung wird durch Zugabe von Ethanol gefällt, der Niederschlag abgesaugt, mit Ethanol und Methanol gewaschen und getrocknet. Es werden so 125 g Produkt erhalten, das überwiegend aus dem Stoff obiger Formel besteht und dessen Struktur mittels IR- und NMR-Spektrum sowie der Elementaranalyse bewiesen wird.

berechnet für $C_7H_{10}N_8O_5$:
C 29,4, H 3,5, N 39,2%
gefunden
C 29,2, H 3,8, N 39,0%

Beispiel 5

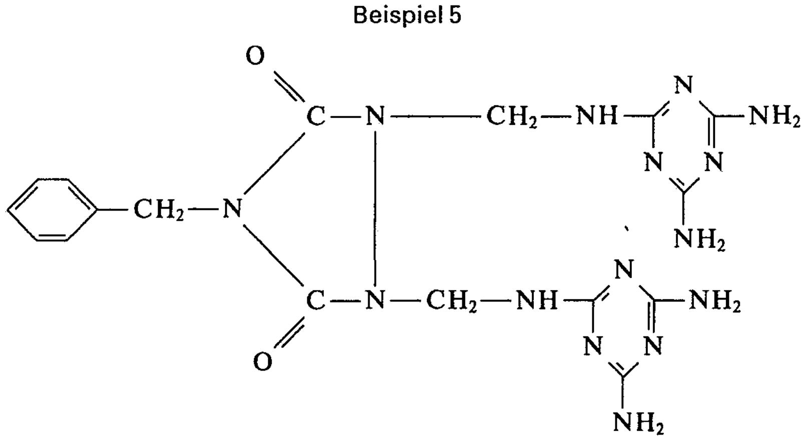

32,8 g einer 37%igen wäßrigen Formaldehydlösung, welche 0,1 ml einer 45%igen Natronlauge enthält, wird bei Raumtemperatur mit 38,2 g 4-Benzyl-triazolidin-3,5-dion versetzt und eine Stunde bei

50°C gerührt. Die klare Lösung wird mit 500 ml Wasser verdünnt, mit 50,4 g Melamin versetzt, auf 95°C aufgeheizt und 3 Stunden bei 95°C zur Vervollständigung der Reaktion gerührt. Der entstandene Niederschlag wird heiß abgesaugt, mit Wasser gewaschen und bei 100°C/40 mbar getrocknet. Es werden 92 g Produkt erhalten, dessen obige Formel mittels IR-Spektrum und der Elementaranalyse bewiesen wird.

berechnet für $C_{17}H_{21}N_{15}O_2$:
C 45,7, H 4,5, N 45%
gefunden
C 45,3, H 4,3, N 45,4%

Beispiel 6

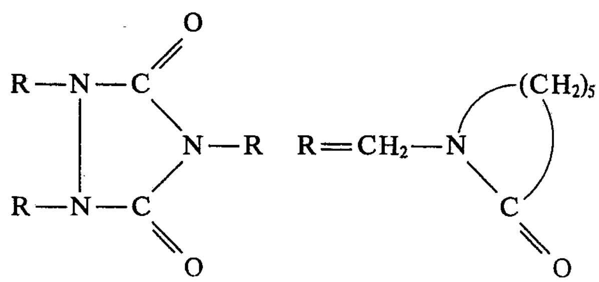

45 g einer wäßrigen 37%igen Formaldehydlösung werden bei Temperaturen <50°C portionsweise mit 20,2 g Triazolidin-3,5-dion versetzt und eine Stunde bei 50°C gerührt. Die erhaltene klare Lösung wird durch Anlegen von Vakuum eingeengt und der farblose viskose Rückstand mit 67,8 g ε-Caprolactam, 250 ml Toluol und 0,5 g p-Toluolsulfonsäure versetzt. Die erhaltene Mischung wird 8 Stunden auf Rückfluß erhitzt, wobei die letzten Reste Wasser sowie das bei der Kondensation entstehende Wasser mittels Wasserabscheider laufend entfernt wird. Die Toluollösung wird nach dem Abkühlen auf Raumtemperatur von wenig Rückstand dekantiert und mit 100 ml Wasser ausgewaschen. Nach dem Trocknen über Natriumsulfat wird die organische Phase am Rotationsverdampfer eingeengt und bei 0,5 mbar und 60°C bis zur Gewichtskonstanz getrocknet. Es verbleiben 62,4 g einer dünnschichtchromatographisch fast reinen Verbindung in Form eines farblosen Öles, welches nach einigen Stunden zu kristallisieren beginnt und dessen Struktur mittels IR- und NMR-Spektrum sowie der Elementaranalyse bestätigt wird.

berechnet für $C_{23}H_{36}N_6O_5$:
C 57,9, H 7,56, N 17,6%
gefunden
C 57,6, H 7,5, N 17,5%

Beispiel 7

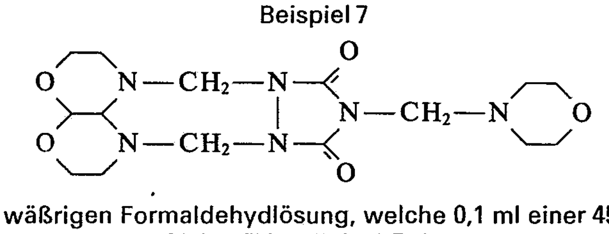

44,8 g einer 37%igen wäßrigen Formaldehydlösung, welche 0,1 ml einer 45%igen Natronlauge enthält, wird bei Raumtemperatur mit 20,2 g Triazolidin-3,5-dion versetzt und eine Stunde bei 50°C gerührt. Durch Anlegen von Vakuum wird die klare Lösung aufkonzentriert, mit 80 g Morpholin, 250 ml Toluol und 0,5 g p-Toluolsulfonsäure versetzt und 8 Stunden bei Rückfluß gerührt, wobei das restliche und das bei der Reaktion entstehende Wasser über einen Wasserabscheider entfernt wird. Die kalte Toluollösung wird mit Wasser ausgewaschen und nach dem Trocknen über $Na_2SO_4$ am Rotationsverdampfer eingeengt. Der verbleibende ölige Rückstand kristallisiert nach einigen Stunden durch und kann nach Zugabe von wenig Cyclohexan abgesaugt werden. Es werden 36 g einer farblosen kristallinen Verbindung erhalten, die nach dem Umkristallisieren aus Cyclohexan einen Fp. von 118—119°C aufweist und dessen obige Formel mittels IR- und NMR-Spektrum sowie der Elementaranalyse bestätigt wird.

berechnet für $C_{17}H_{30}N_6O_5$:
C 51,2, H 7,54, N 21,1%
gefunden
C 51,4, H 7,4, N 21,1%

Beispiel 8

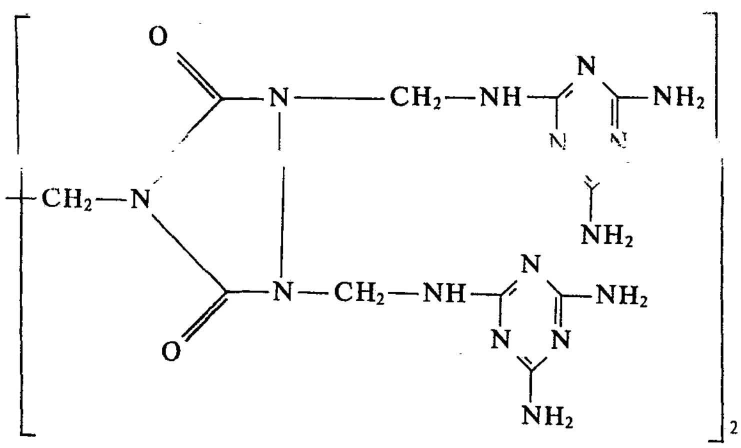

33 g einer 37%igen wäßrigen Formaldehydlösung, welche 0,1 ml einer 45%igen Natronlauge enthält, wird bei Raumtemperatur mit 22,8 g 1,2-Ethandiyl-4,4'-bis-1,2,4-triazolidin-3,5-dion versetzt und nach Beendigung der leicht exothermen Reaktion eine Stunde bei 50°C gerührt. Die klare Lösung wird mit 500 ml Wasser verdünnt, mit 50,4 g Melamin versetzt, auf 95°C aufgeheizt und 2 Stunden bei 95°C gerührt. Der entstandene Niederschlag wird heiß abgesaugt, mit Wasser gewaschen und bei 100°C/35 mbar getrocknet. Es werden 76,2 g Produkt vom Fp. >260°C erhalten, dessen obige Formel mittels IR-Spektrum und der Elementaranalyse bewiesen wird.

berechnet für $C_{22}H_{16}N_{30}O_4$:
C 33,8, H 4,12, N 54,8%
gefunden
C 34,1, H 4,3, N 54,5%

Mit dieser Verbindung werden bei Flammschutztests an thermoplastischen Polyamid-6,6 ähnliche Ergebnisse erzielt wie bei Beispiel 1b.

Beispiel 9

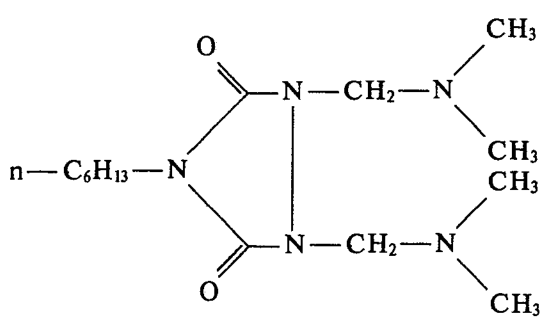

Zu 33 g einer 37%igen wäßrigen Formaldehydlösung, welche 0,1 g Soda enthält, werden bei Raumtemperatur 37 g 4-(n-Hexyl)-triazolidin-3,5-dion zugegeben. Nach Beendigung der exothermen Reaktion werden 40 g einer 52%igen wäßrigen Dimethylaminlösung zugetropft und 2 Stunden bei 80°C gerührt. Nach dem Abdestillieren des Wassers wird über Kieselgel filtriert, die flüchtigen Bestandteile, zuletzt bei 50°C/0,3 mbar entfernt. Es werden 29,1 g eines braunen Öles erhalten, dessen IR- und NMR-Spektren sowie die Elementaranalyse die obige Formel bestätigen.

berechnet für $C_{14}H_{29}N_5O_2$:
C 56,2, H 9,81, N 23,4%
gefunden
C 55,8, H 9,5, N 23,7%

Beispiel 10

In einem 6-l-Dreihalskolben, der mit Rührer, Thermometer, Tropftrichter und Destillationseinrich-

tung ausgestattet ist, werden 3 kg Sulfolan und 1,18 kg an der Luft getrocknetes Hydrazodicarbonamid innerhalb 2,5 Stunden auf 200°C aufgeheizt, wobei zu Beginn der Ammoniakentwicklung bei 150—160°C leichtes Vakuum angelegt wird. Dann wird die Temperatur innerhalb einer Stunde auf 210°C erhöht. Nach ca. 1,5 Stunden entsteht eine klare Lösung, die noch ca. 3,5 Stunden bei 210°C und 200 mbar bis zur Beendigung der Reaktion weitergerührt wird. Das restliche Ammoniak wird nach Abkühlen auf 180° bei 40 bis 80 mbar entfernt. Zu der abkühlenden Lösung werden bei Normaldruck 0,8 kg Toluol so zugetropft, daß praktisch kein Toluol abdestilliert. Das auskristallisierte, fast reine 1,2,4-Triazolidin-3,5-dion wird nach dem Abkühlen auf Raumtemperatur abgesaugt und mit Toluol gewaschen. Es werden 0,87 kg (= 86,2% der theoretischen Ausbeute) getrocknetes 1,2,4-Triazolidin-3,5-dion mit einer Reinheit von 97,5%, bestimmt durch Titration mit n/10 Natronlauge gegen Phenolphthalein, erhalten.

Beispiel 11

53,5 g Benzylamin und 59 g Hydrazodicarbonamid werden in 100 ml N-Methylpyrrolidon 4 Stunden bei 175°C und 5 Stunden bei 200°C gerührt. Anschließend destilliert man das Lösungsmittel im Vakuum ab, verreibt den Rückstand mit 50 ml 10%iger Natronlauge und saugt vom Rückstand ab. Das Filtrat wird mit 10%iger Salzsäure neutralisiert. Dabei entsteht ein Niederschlag, der abgesaugt und mit Wasser gewaschen wird. Man erhält 67 g (70% der theoretischen Ausbeute) 4-Benzyl-1,2,4-triazolidin-3,5-dion als farblose Kristalle vom Fp. 185—188°C ($CH_3CN$).

Beispiel 12

600 g Hydrazodicarbonamid und 150 g Ethylendiamin werden in 500 ml N-Methylpyrrolidon 4 Stunden bei 175°C und 20 Stunden bei 200°C gerührt. Beim Abkühlen fällt ein Niederschlag aus, der abgesaugt und mit Ethanol gewaschen wird. Man erhält 462 g (80% der theoretischen Ausbeute) 1,2-Ethandiyl-4,4'-bis-1,2,4-triazolidin-3,5-dion als farblose Kristalle vom Fp. >330°C.

Beispiel 13

60 g Hydrazodicarbonamid und 56 g n-Hexylamin werden in 100 ml N-Methylpyrrolidon 6 Stunden bei 150°C, 20 Stunden bei 175°C und 20 Stunden bei 180°C gerührt. Anschließend wird das Lösungsmittel im Vakuum abdestilliert und der Rückstand mit 100 ml 10%iger Natronlauge verrieben. Man saugt vom Niederschlag ab und neutralisiert das Filtrat mit 10%iger Salzsäure. Dabei fällt ein Niederschlag aus, der abgesaugt, mit Wasser gewaschen und im Vakuum getrocknet wird. Man erhält 75 g (81% der theoretischen Ausbeute) 4-(n-Hexyl)-1,2,4-triazolidin-3,5-dion als farblose Kristalle vom Fp. 144—145°C.

**Patentansprüche**

1. Triazolidin-3,5-dion-Formaldehyd-Amin-Kondensate der Formel

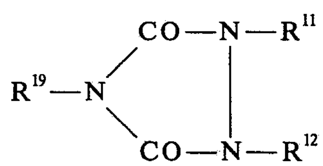

in der

$R^{11}$ und $R^{12}$ unabhängig voneinander Wasserstoff, $C_2-C_8$-Alkylcarbonyl oder die Gruppe

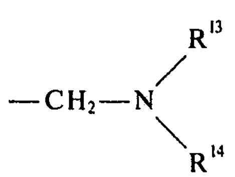

bedeuten wobei

$R^{13}$ für Wasserstoff, $C_1-C_8$-Alkyl, $C_5-C_6$-Cycloalkyl, Benzyl oder Phenyl steht,

$R^{14}$ $C_1-C_8$-Alkyl, $C_2-C_8$-Alkenyl, Benzyl, Phenyl, einen ein oder mehrere Stickstoff-, Sauerstoff- oder Schwefelatome enthaltenden heterocyclischen Rest, $C_2-C_8$-Alkylcarbonyl, $C_3-C_8$-Alkenylcarbonyl oder $C_2-C_8$-Alkoxycarbonyl bedeutet und in der weiterhin

$R^{13}$ und $R^{14}$ gemeinsam mit dem Stickstoffatom, das sie substituieren, einen gegebenenfalls weitere Stickstoff-, Sauerstoff- oder Schwefelatome enthaltenden Stickstoffheterocyclus bedeuten können, und

$R^{19}$ für $C_1-C_{20}$-Alkyl, $C_5-C_6$-Cycloalkyl, Benzyl, Phenyl, $C_2-C_8$-Alkylcarbonyl oder die Gruppe

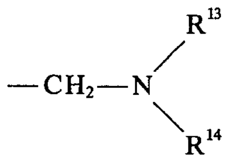

steht, in der

$R^{13}$ und $R^{14}$ die angegebene Bedeutung haben

oder für die Gruppe

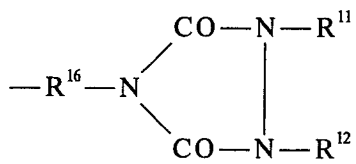

steht, in der

$R^{16}$ $C_1-C_{12}$-Alkylen, Phenylen, Toluylen oder eine der Gruppen

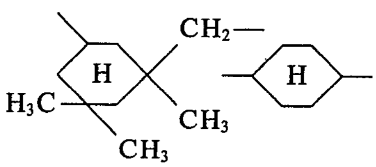

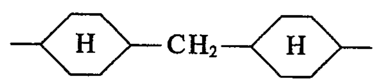

oder

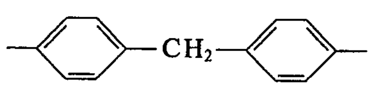

bedeutet, und

$R^{11}$ und $R^{12}$ die obengenannte Bedeutung haben,

oder für die Gruppe

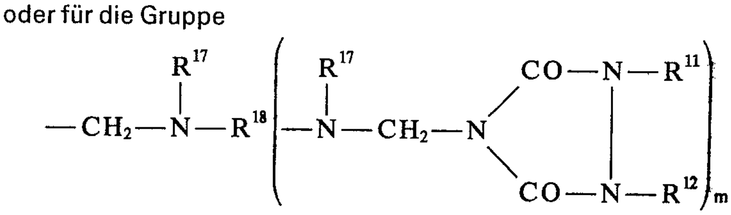

steht, in der

$R^{11}$ und $R^{12}$ die obengenannte Bedeutung haben,

$R^{17}$ für Wasserstoff, $C_1-C_4$-Alkyl, Cyclohexyl, Benzyl, Phenyl, einen ein oder mehrere Stickstoff-, Sauerstoff- oder Schwefelatome enthaltenden heterocyclischen Rest oder die Gruppe

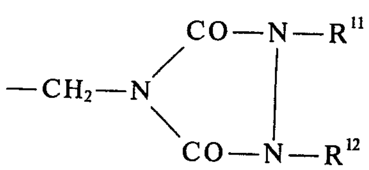

steht,

$R^{18}$ ein (m+1)-wertiger aliphatischer Rest ($C_2-C_8$), Cyclohexanrest, Benzolrest oder ein ein oder mehrere Stickstoff-, Sauerstoff- oder Schwefelatome enthaltender heterocyclischer Rest ist,
m die Zahl 0, 1 oder 2 bedeutet, wobei für den Fall, daß m = 1 ist, $R^{18}$ auch die Carbonylgruppe sein kann,

und wobei die Reste $R^{11}$, $R^{12}$ und $R^{19}$ mindestens eine aus der Formaldehyd-Amin-Kondensation hervorgegangene Gruppe

$>N-CH_2-N<$

enthalten.

2. Triazolidin-3,5-dion-Formaldehyd-Amin-Kondensate nach Anspruch 1 der Formel

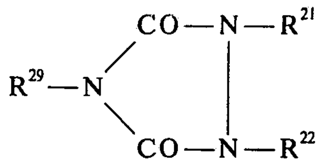

in der

$R^{21}$ und $R^{22}$ unabhängig voneinander Wasserstoff, $C_2-C_4$-Alkylcarbonyl oder die Gruppe

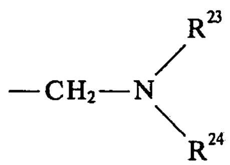

bedeuten, in der

$R^{23}$ für Wasserstoff, $C_1-C_4$-Alkyl und
$R^{24}$ für $C_1-C_4$-Alkyl, einen ein oder mehrere Stickstoff-, Sauerstoff- oder Schwefelatome enthaltenden heterocyclischen Rest, $C_2-C_4$-Alkylcarbonyl, $C_3-C_4$-Alkenylcarbonyl stehen und wobei weiterhin
$R^{23}$ und $R^{24}$ gemeinsam mit dem Stickstoffatomen, das sie substituieren, einen gegebenenfalls weitere Stickstoff-, Sauerstoff- oder Schwefelatome enthaltenden Stickstoffheterocyclus bilden können, und

$R^{29}$ $C_1-C_4$-Alkyl, Cyclohexyl, Benzyl, Phenyl, $C_2-C_4$-Alkylcarbonyl oder die Gruppe

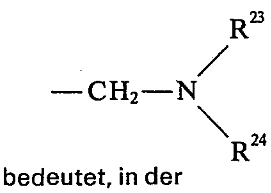

bedeutet, in der

$R^{23}$ und $R^{24}$ die oben angegebene Bedeutung haben,

wobei die Reste $R^{21}$, $R^{22}$ und $R^{23}$ mindestens eine aus der Formaldehyd-Amin-Kondensation hervorgegangene Gruppe

$>N-CH_2-N<$

enthalten.

3. Triazolidin-3,5-dion-Formaldehyd-Amin-Kondensate nach Anspruch 1 der Formel

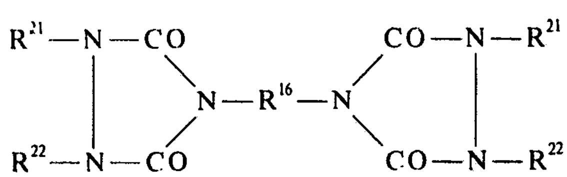

in der

$R^{21}$ und $R^{22}$ den in Anspruch 2 genannten Bedeutungsumfang haben und
$R^{16}$ $C_1-C_{12}$-Alkylen, Phenylen, Toluylen oder eine der Gruppen

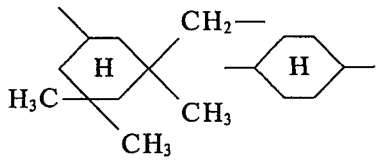

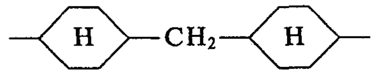

oder

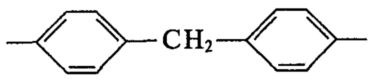

bedeutet, und wobei die Reste $R^{21}$ und $R^{22}$ mindestens eine aus der Formaldehyd-Amin-Kondensation hervorgegangene Gruppe

$>N-CH_2-N<$

enthalten.

4. Triazolidin-3,5-dion-Formaldehyd-Amin-Kondensate der Formel

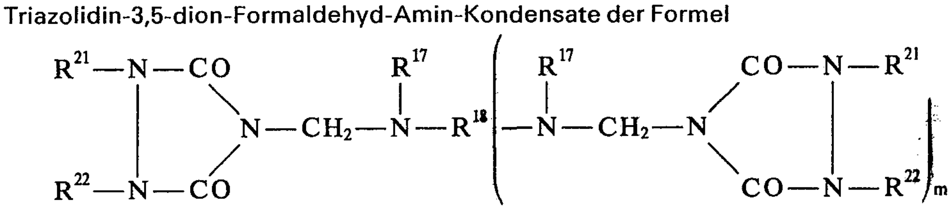

in der

$R^{21}$ und $R^{22}$ den in Anspruch 2 genannten Bedeutungsumfang haben,
$R^{17}$ für Wasserstoff, $C_1-C_4$-Alkyl, Cyclohexyl, Benzyl, Phenyl, einen ein oder mehrere Stickstoff-, Sauerstoff- oder Schwefelatome enthaltender heterocyclischen Rest oder die Gruppe

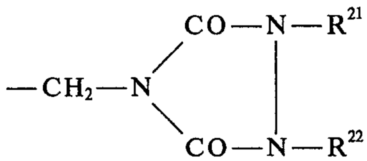

steht, in der

$R^{21}$ und $R^{22}$ den in Anspruch 2 genannten Bedeutungsumfang haben,

$R^{18}$ ein (m+1)-wertiger aliphatischer Rest ($C_2-C_8$), Cyclohexanrest, Benzolrest oder ein ein oder mehrere Stickstoff-, Sauerstoff- oder Schwefelatome enthaltender heterocyclischer Rest ist und
m die Zahl 0, 1 oder 2 bedeutet, wobei für den Fall, daß m=1 ist, $R^{18}$ auch die Carbonylgruppe sein kann

und wobei die Reste $R^{21}$, $R^{22}$, $R^{17}$ und $R^{18}$ mindestens eine aus der Formaldehyd-Amin-Kondensation hervorgegangene Gruppe

$>N-CH_2-N<$

enthalten.

5. Verfahren zur Herstellung von Triazolidin-3,5-dion-Formaldehyd-Amin-Kondensaten nach Anspruch 1, dadurch gekennzeichnet, daß man Triazolidin-3,5-dione der Formel

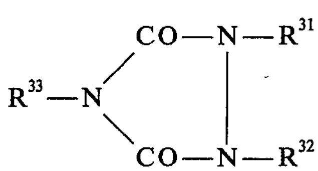

in der

$R^{31}$ und $R^{32}$ unabhängig voneinander Wasserstoff oder $C_2-C_8$-Alkylcarbonyl bedeuten und
$R^{33}$ Wasserstoff, Alkyl ($C_1-C_{20}$), Cycloalkyl ($C_5-C_6$), Benzyl, Phenyl, Alkylcarbonyl ($C_2-C_8$) oder die Gruppe

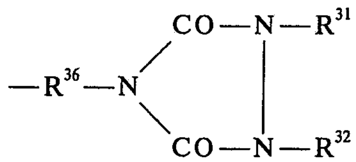

bedeutet, in der

$R^{31}$ und $R^{32}$ die obengenannte Bedeutung haben,
$R^{36}$ $C_1-C_{12}$-Alkylen, Phenylen, Toluylen oder eine der Gruppen

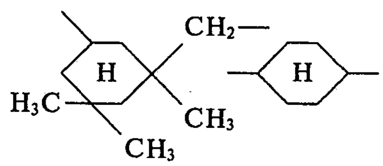

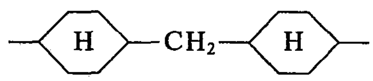

oder

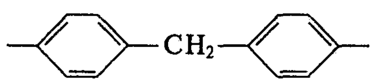

bedeutet, wobei mindestens einer der Reste $R^{31}$, $R^{32}$ und $R^{33}$ Wasserstoff bedeutet, mit Formaldehyd und Stickstoffverbindungen der Formel

H-X

in der

X für

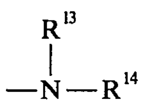 oder 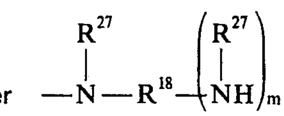

steht, worin

$R^{13}$ für Wasserstoff, $C_1-C_8$-Alkyl, $C_5-C_6$-Cycloalkyl, Benzyl oder Phenyl steht,
$R^{14}$ $C_1-C_8$-Alkyl, $C_2-C_8$-Alkenyl, Benzyl, Phenyl, einen ein oder mehrere Stickstoff-, Sauerstoff- oder Schwefelatome enthaltenden heterocyclischen Rest, $C_2-C_8$-Alkylcarbonyl, $C_3-C_8$-Alkenylcarbonyl oder $C_2-C_8$-Alkoxycarbonyl bedeutet und in der weiterhin
$R^{13}$ und $R^{14}$ gemeinsam mit dem Stickstoffatom, das sie substituieren, einen gegebenenfalls weitere Stickstoff-, Sauerstoff- oder Schwefelatome enthaltenden Stickstoffheterocyclus bedeuten können, und
$R^{18}$ ein (m+1)-wertiger aliphatischer Rest ($C_2-C_8$), Cyclohexanrest, Benzolrest oder ein ein oder mehrere Stickstoff-, Sauerstoff- oder Schwefelatome enthaltender heterocyclischer Rest ist und
m die Zahl 0, 1 oder 2 bedeutet, wobei für den Fall, daß m=1 ist, $R^{18}$ auch die Carbonylgruppe sein kann, und
$R^{27}$ Wasserstoff, $C_1-C_4$-Alkyl, Cyclohexyl, Benzyl, Phenyl oder einen ein oder mehrere Stickstoff-, Sauerstoff- oder Schwefelatome enthaltenden heterocyclischen Rest bedeutet,

bei Temperaturen von 10 bis 180° C, gegebenenfalls in Gegenwart eines Katalysators und gegebenenfalls in Gegenwart eines Lösungsmittels umsetzt.

6. Verfahren nach Anspruch 5, dadurch gekennzeichnet, daß in einer ersten Verfahrensstufe zunächst die Triazolidin-3,5-dione mit dem Formaldehyd und dann in einer zweiten Verfahrensstufe weiter mit der Stickstoffverbindung umgesetzt werden.

**Claims**

1. Triazolidine-3,5-dione/formaldehyde/amine condensates of the formula

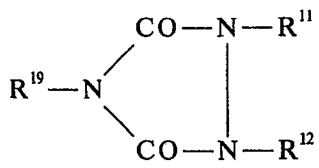

in which

$R^{11}$ and $R^{12}$ independently of one another denote hydrogen, $C_2-C_8$-alkylcarbonyl or the group

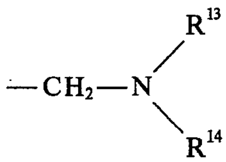

where in

$R^{13}$ represents hydrogen, $C_1-C_8$-alkyl, $C_5-C_6$-cycloalkyl, benzyl or phenyl,
$R^{14}$ denotes $C_1-C_8$-alkyl, $C_2-C_8$-alkenyl, benzyl, phenyl, a heterocyclic radical containing one or more nitrogen, oxygen or sulphur atoms, $C_2-C_8$-alkylcarbonyl, $C_3-C_8$-alkenylcarbonyl or $C_2-C_8$-alkoxycarbonyl, and in which furthermore
$R^{13}$ and $R^{14}$, together with the nitrogen atom on which they are substituents, can denote a nitrogen heterocyclic radical which optionally contains other nitrogen, oxygen or sulphur atoms, and
$R^{19}$ represents $C_1-C_{20}$-alkyl, $C_5-C_6$-cycloalkyl, benzyl, phenyl, $C_2-C_8$-alkylcarbonyl, or the group

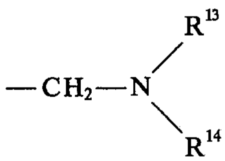

in which

$R^{13}$ and $R^{14}$ have the meaning given, or represents the group

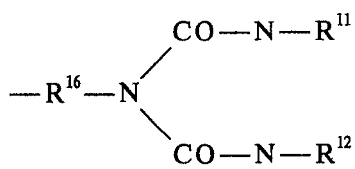

in which

$R^{16}$ denotes $C_1-C_{12}$-alkylene, phenylene, toluylene or one of the groups

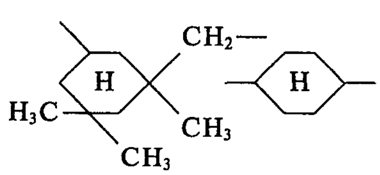

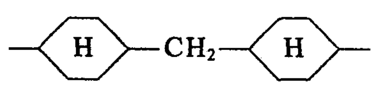

or

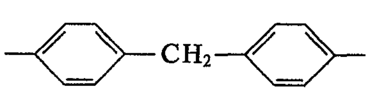

and

$R^{11}$ and $R^{12}$ have the abovementioned meaning, or represents the group

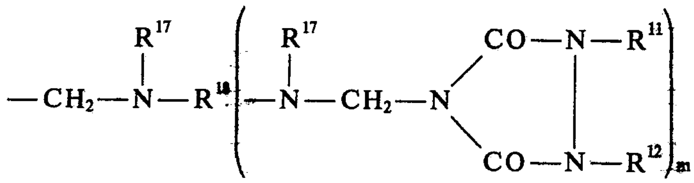

in which

$R^{11}$ and $R^{12}$ have the abovementioned meaning,
$R^{17}$ represents hydrogen, $C_1-C_4$-alkyl, cyclohexyl, benzyl, phenyl, a heterocyclic radical containing one or more nitrogen, oxygen or sulphur atoms, or the group

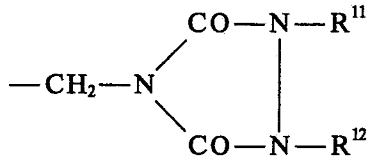

$R^{18}$ is a (m+1)-valent aliphatic radical ($C_2-C_8$), cyclohexane radical, benzene radical or a heterocyclic radical containing one or more nitrogen, oxygen or sulphur atoms,
m denotes the number 0, 1 or 2, it also being possible for $R^{18}$ to be the carbonyl group if m is 1,

and wherein
the radicals $R^{11}$, $R^{12}$ and $R^{19}$ contain at least one group

$$>N-CH_2-N<$$

arising from the formaldehyde/amine condensation.

2. Triazolidine-3,5-dione/formaldehyde/amine condensates according to claim 1, of the formula

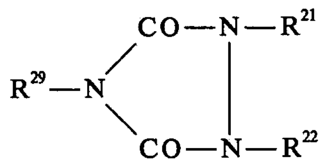

in which

$R^{21}$ and $R^{22}$ independently of one another denote hydrogen, $C_2-C_4$-alkylcarbonyl or the group

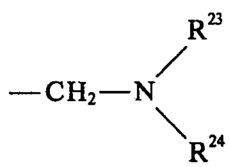

in which

$R^{23}$ represents hydrogen or $C_1-C_4$-alkyl and
$R^{24}$ represents $C_1-C_4$-alkyl, a heterocyclic radical containing one or more nitrogen, oxygen or sulphur atoms, $C_2-C_4$-alkylcarbonyl or $C_3-C_4$-alkenylcarbonyl, and wherein furthermore
$R^{23}$ and $R^{24}$, together with the nitrogen atom on which they are substituents, can form a nitrogen heterocyclic radical which optionally contains other nitrogen, oxygen or sulphur atoms, and

$R^{29}$ denotes $C_1-C_4$-alkyl, cyclohexyl, benzyl, phenyl, $C_2-C_4$-alkylcarbonyl or the group

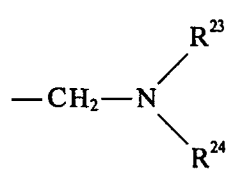

in which

$R^{23}$ and $R^{24}$ have the abovementioned meaning,

the radicals $R^{21}$, $R^{22}$ and $R^{23}$ containing at least one group

$$>N-CH_2-N<$$

arising from the formaldehyde/amine condensation.

3. Triazolidine-3,5-dione/formaldehyde/amine condensates according to claim 1, of the formula

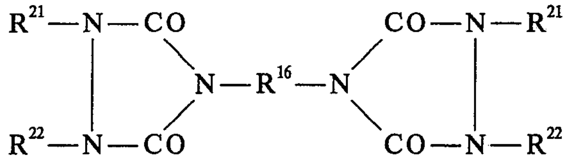

in which

$R^{21}$ and $R^{22}$ have the scope of meaning given in claim 2 and
$R^{16}$ denotes $C_1-C_{12}$-alkylene, phenylene, toluylene or one of the groups

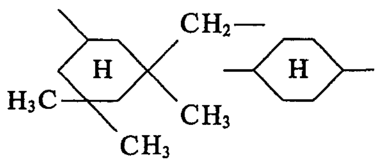

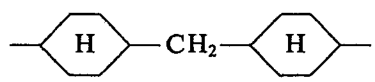

or

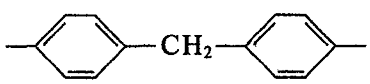

and wherein the radicals $R^{21}$ and $R^{22}$ contain at least one group

$$>N-CH_2-N<$$

arising from the formaldehyde/amine condensation.

4. Triazolidine-3,5-dione/formaldehyde/amine condensates of the formula

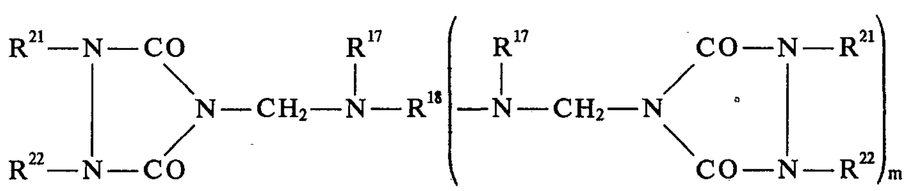

in which

$R^{21}$ and $R^{22}$ have the scope of meaning given in claim 2,
$R^{17}$ represents hydrogen, $C_1-C_4$-alkyl, cyclohexyl, benzyl, phenyl, a heterocyclic radical containing one or more nitrogen, oxygen or sulphur atoms, or the group

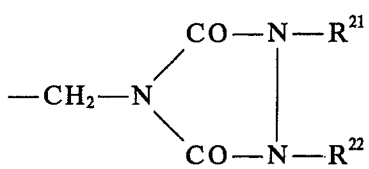

in which

$R^{21}$ and $R^{22}$ have the scope of meaning given in claim 2,

$R^{18}$ is a (m+1)-valent aliphatic radical ($C_2-C_8$), cyclohexane radical, benzene radical or a heterocyclic radical containing one or more nitrogen, oxygen or sulphur atoms, and
m denotes the number 0, 1 or 2, it also being possible for $R^{18}$ to be the carbonyl group if m is 1,

and wherein the radicals $R^{21}$, $R^{22}$, $R^{17}$ and $R^{18}$ contain at least one group

$$>N-CH_2-N<$$

arising from the formaldehyde/amine condensation.

5. Process for the preparation of triazolidine-3,5-dione/formaldehyde/amine condensates according to claim 1, characterised in that triazolidine-3,5-diones of the formula

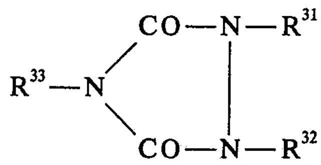

in which

$R^{31}$ and $R^{32}$ independently of one another denote hydrogen or $C_2-C_8$-alkylcarbonyl and

$R^{33}$ denotes hydrogen, alkyl ($C_1-C_{20}$), cycloalkyl ($C_5-C_6$), benzyl, phenyl, alkylcarbonyl ($C_2-C_8$) or the group

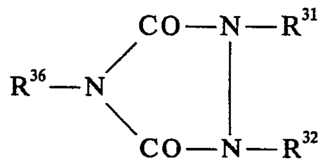

in which

$R^{31}$ and $R^{32}$ have the abovementiones meaning,

$R^{36}$ denotes $C_1-C_{12}$-alkylene, phenylene, toluylene or one of the groups

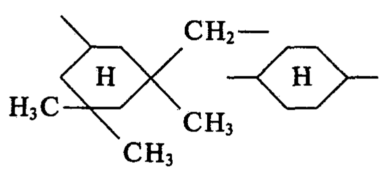

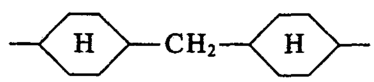

or

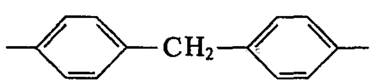

wherein

at least one of the radicals $R^{31}$, $R^{32}$ and $R^{33}$ denotes hydrogen,

are reacted with formaldehyde and nitrogen compounds of the formula

$$H-X$$

in which

X represents

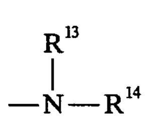 oder 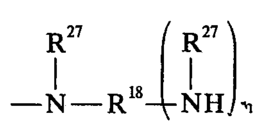

wherein

$R^{13}$ represents hydrogen, $C_1-C_8$-alkyl, $C_5-C_6$-cycloalkyl, benzyl or phenyl,

$R^{14}$ denotes $C_1-C_8$-alkyl, $C_2-C_8$-alkenyl, benzyl, phenyl, a heterocyclic radical containing one or more nitrogen, oxygen or sulphur atoms, $C_2-C_8$-alkylcarbonyl, $C_3-C_8$-alkenylcarbonyl or

$C_2-C_8$-alkoxycarbonyl and in which furthermore

$R^{13}$ and $R^{14}$, together with the nitrogen atom on which they are substituents, can denote a nitrogen heterocyclic radical which optionally contains other nitrogen, oxygen or sulphur atoms, and

$R^{18}$ is a (m+1)-valent aliphatic radical ($C_2-C_8$), cyclohexane radical, benzene radical or a heterocyclic radical containing one or more nitrogen, oxygen or sulphur atoms and

m denotes the number 0, 1 or 2, it also being possible for $R^{18}$ to be the carbonyl group if m is 1, and

$R^{27}$ denotes hydrogen, $C_1-C_4$-alkyl, cyclohexyl, benzyl, phenyl or a heterocyclic radical containing one or more nitrogen, oxygen, or sulphur atoms,

at temperatures of 10 to 180°C, if appropriate in the presence of a catalyst and if appropriate in the presence of a solvent.

6. Process according to claim 5, characterised in that the triazolidine-3,5-diones are first reacted with the formaldehyde in a first process stage and the product is then reacted further with the nitrogen compound in a second process stage.

## Revendications

1. Produits de condensation triazolidine-3,5-dione-formaldéhyde-amine de formule

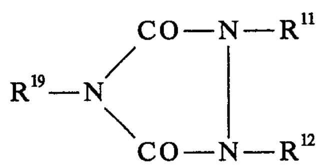

dans laquelle

$R^{11}$ et $R^{12}$ représentent, indépendamment l'un de l'autre, l'hydrogène, un groupe alkylcarbonyle en $C_2$ à $C_8$ ou le groupe

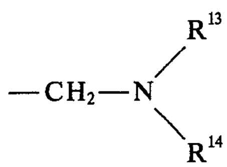

où

$R^{13}$ désigne l'hydrogène, un radical alkyle en $C_1$ à $C_8$, cycloalkyle en $C_5$ ou $C_6$, benzyle ou phényle,

$R^{14}$ est un radical alkyle en $C_1$ à $C_8$, alcényle en $C_2$ à $C_8$, benzyle, phényle, un reste hétérocyclique contenant un ou plusieurs atomes d'azote, d'oxygène ou de soufre, un reste alkylcarbonyle en $C_2$ à $C_8$, alcénylcarbonyle en $C_3$ à $C_8$ ou alkoxycarbonyle en $C_2$ à $C_8$ et, en outre,

$R^{13}$ et $R^{14}$ forment conjointement avec l'atome d'azote qu'ils substituent un hétérocycle azoté contenant éventuellement d'autres atomes d'azote, d'oxygène ou de soufre, et

$R^{19}$ est un radical alkyle en $C_1$ à $C_{20}$, cycloalkyle en $C_5$ ou $C_6$, benzyle, phényle, alkylcarbonyle en $C_2$ à $C_8$ ou le groupe

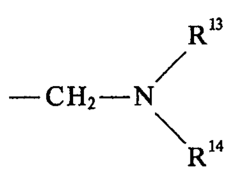

dans lequel

$R^{13}$ et $R^{14}$ ont la définition indiquée ci-dessus, ou le groupe

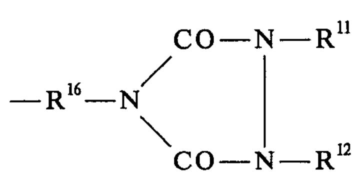

dans lequel

$R^{16}$ est un radical alkylène en $C_1$ à $C_{12}$, phénylène, toluylène ou l'un des groupes

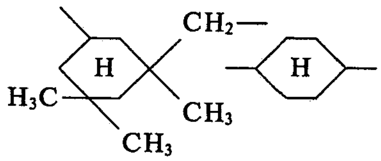

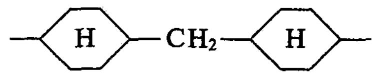

ou

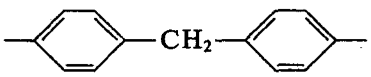

et

$R^{11}$ et $R^{12}$ ont la définition indiquée ci-dessus,

ou bien le groupe

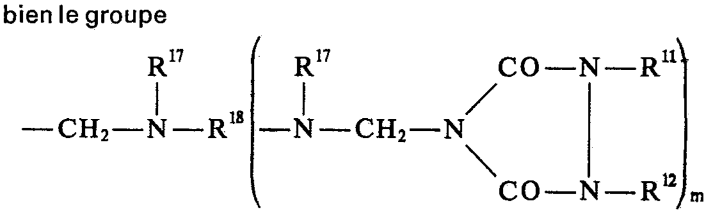

dans lequel

$R^{11}$ et $R^{12}$ ont la définition indiquée ci-dessus,

$R^{17}$ représente l'hydrogène, un radical alkyle en $C_1$ à $C_4$, cyclohexyle, benzyle, phényle, un reste hétérocyclique comportant un ou plusieurs atomes d'azote, d'oxygène ou de soufre, ou le groupe

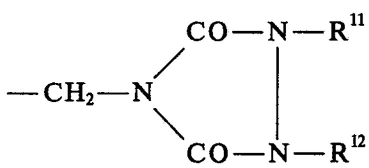

$R^{18}$ est un reste aliphatique de valeur (m + 1) (en $C_2$ à $C_8$), un reste cyclohexane, un reste benzène ou un reste hétérocyclique comprenant un ou plusieurs atomes d'azote, d'oxygène ou de soufre,

m représente le nombre 0, 1 ou 2, et au cas où m est égal à 1, $R^{18}$ peut aussi être le groupe carbonyle,

et les restes $R^{11}$, $R^{12}$ et $R^{19}$ comprennent au moins un groupe

$>N-CH_2-N<$

provenant de la condensation formaldéhyde-amine.

2. Produits de condensation triazolidine-3,5-dione-formaldéhyde-amine suivant la revendication 1, de formule

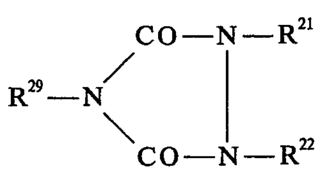

dans laquelle

$R^{21}$ et $R^{22}$ représentent, indépendamment l'un de l'autre, l'hydrogène, un groupe alkylcarbonyle en $C_2$ à $C_4$ ou le groupe de formule

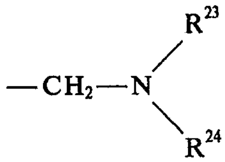

dans laquelle

$R^{23}$ représente l'hydrogène, un groupe alkyle en $C_1$ à $C_4$ et
$R^{24}$ est un groupe alkyle en $C_1$ à $C_4$, un reste hétérocyclique comportant un ou plusieurs atomes d'azote, d'oxygène ou de soufre, un groupe alkylcarbonyle en $C_2$ à $C_4$, un groupe alcénylcarbonyle en $C_3$ ou $C_4$, et en outre
$R^{23}$ et $R^{24}$ peuvent former conjointement avec l'atome d'azote qu'ils substituent un hétérocycle azoté contenant éventuellement d'autres atomes d'azote, d'oxygène ou de soufre, et

$R^{29}$ est un groupe alkyle en $C_1$ à $C_4$, cyclohexyle, benzyle, phényle, alkylcarbonyle en $C_2$ à $C_4$ ou le groupe

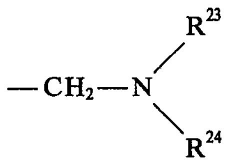

dans lequel

$R^{23}$ et $R^{24}$ ont la définition indiquée ci-dessus,

les restes $R^{21}$, $R^{22}$ et $R^{23}$ comportant au moins un groupe

$>N-CH_2-N<$

provenant de la condensation formaldéhyde-amine.

3. Produits de condensation triazolidine-3,5-dione-formaldéhyde-amine suivant la revendication 1, de formule

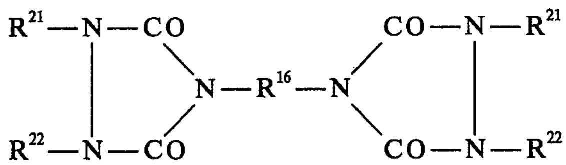

dans laquelle

$R^{21}$ et $R^{22}$ ont la définition mentionnée dans la revendication 2 et
$R^{16}$ est un groupe alkylène en $C_1$ à $C_{12}$, phénylène, toluylène ou l'un des groupes

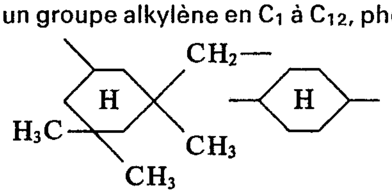

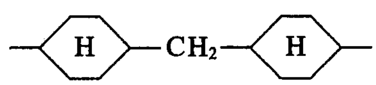

ou

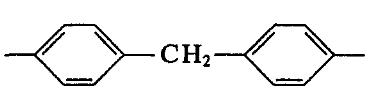

et les restes $R^{21}$ et $R^{22}$ comprennent au moins un groupe

$>N-CH_2-N<$

provenant de la condensation formaldéhyde-amine.

4. Produits de condensation triazolidine-3,5-dione-formaldéhyde-amine de formule

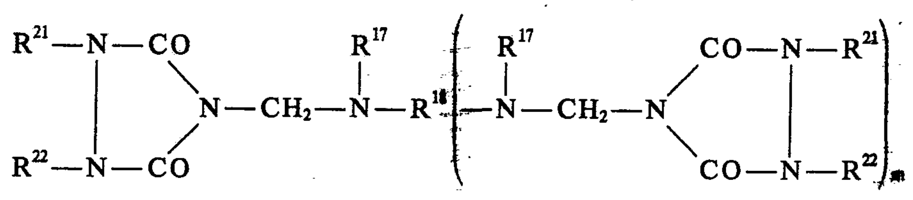

dans laquelle

$R^{21}$ et $R^{22}$ ont la définition donnée dans la revendication 2,

$R^{17}$ représente l'hydrogène, un groupe alkyle en $C_1$ à $C_4$, cyclohexyle, benzyle, phényle, un reste hétérocyclique comportant un ou plusieurs atomes d'azote, d'oxygène ou de soufre ou le groupe

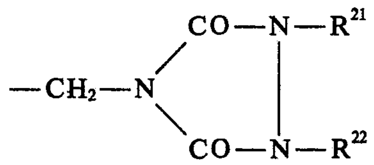

dans lequel

$R^{21}$ et $R^{22}$ ont les définitions mentionnées dans la revendication 2,

$R^{18}$ est un reste aliphatique de valence $(m+1)$ (en $C_2$ à $C_8$), un reste cyclohexane, un reste benzène ou un reste hétérocyclique comportant un ou plusieurs atomes d'azote, d'oxygène ou de soufre et

m représente le nombre 0, 1 ou 2, et au cas où m est égal à 1, $R^{18}$ peut également être le groupe carbonyle,

et les restes $R^{21}$, $R^{22}$, $R^{17}$ et $R^{18}$ comportent au moins un groupe ,

$>N-CH_2-N<$

provenant de la condensation formaldéhyde-amine.

5. Procédé de préparation de produits de condensation triazolidine-3,5-dione-formaldéhyde-amine suivant la revendication 1, caractérisé en ce qu'on fait réagir à des températures de 10 à 180°C, éventuellement en présence d'un catalyseur et en la présence éventuelle d'un solvant, des triazolidine-3,5-diones de formule

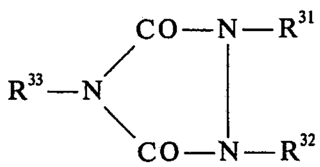

dans laquelle

$R^{31}$ et $R^{32}$ représentent, indépendamment l'un de l'autre, l'hydrogène ou un groupe alkylcarbonyle en $C_2$ à $C_8$ et

$R^{33}$ représente l'hydrogène, un groupe alkyle en $C_1$ à $C_{20}$, cycloalkyle en $C_5$ ou $C_6$, benzyle, phényle, alkylcarbonyle en $C_2$ à $C_8$ ou le groupe

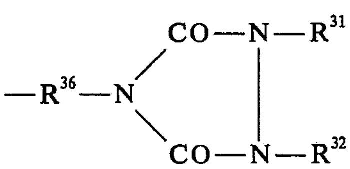

dans lequel

$R^{31}$ et $R^{32}$ ont la définition indiquée ci-dessus,

$R^{36}$ est un groupe alkylène en $C_1$ à $C_{12}$, phénylène, toluylène ou l'un des groupes

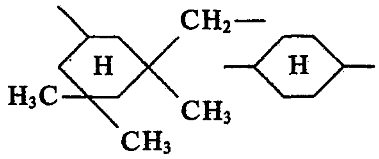

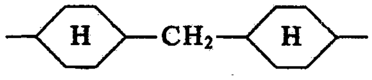

ou

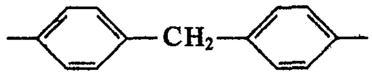

l'un au moins des restes $R^{31}$, $R^{32}$ et $R^{33}$ représentant l'hydrogène, avec le formaldéhyde et des composés azotés de formule

H—X

dans laquelle

X représente

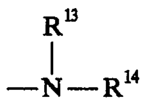

ou

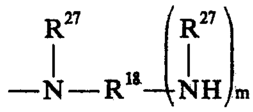

où

$R^{13}$ est l'hydrogène, un groupe alkyle en $C_1$ à $C_8$, cycloalkyle en $C_5$ ou $C_6$, benzène ou phényle,
$R^{14}$ est un groupe alkyle en $C_1$ à $C_8$, alcényle en $C_2$ à $C_8$, benzyle, phényle, un reste hétérocyclique comportant un ou plusieurs atomes d'azote, d'oxygène ou de soufre, un reste alkylcarbonyle en $C_2$ à $C_8$, alcénylcarbonyle en $C_3$ à $C_8$ ou alkoxycarbonyle en $C_2$ à $C_8$ et en outre
$R^{13}$ et $R^{14}$ peuvent former conjointement avec l'atome d'azote qu'ils substituent un hétérocycle azoté comportant éventuellement d'autres atomes d'azote, d'oxygène ou de soufre et
$R^{18}$ est un reste aliphatique en $C_2$ à $C_8$ de valence $(m+1)$, un reste cyclohexane, un reste benzène ou un reste hétérocyclique comportant un ou plusieurs atomes d'azote, d'oxygène ou de soufre et
m représente le nombre 0, 1 ou 2, et au cas où m est égal à 1, $R^{18}$ peut aussi être le groupe carbonyle, et
$R^{27}$ est l'hydrogène, un groupe alkyle en $C_1$ à $C_4$, cyclohexyle, benzyle, phényle ou un reste hétérocyclique comportant un ou plusieurs atomes d'azote, d'oxygène ou de soufre.

6. Procédé suivant la revendication 5, caractérisé en ce que dans une première étape du procédé, on fait réagir tout d'abord les triazolidine-3,5-diones avec le formaldéhyde puis, dans une seconde étape du procédé, on poursuit la réaction avec le composé azoté.